# EUROPÄISCHE PATENTANMELDUNG

(11) **EP 0 807 406 A1**
(43) Veröffentlichungstag der Anmeldung: **19.11.1997**
(21) Anmeldenummer: 97107312.7
(22) Anmeldetag: 02.05.1997
(51) Int. Cl.: A61B 6/02

(54) **Röntgendiagnostikgerät für Tomosynthese**

(30) Priorität: 17.05.1996 DE 19619913
(71) Anmelder: SIEMENS AKTIENGESELLSCHAFT, 80333 München (DE)
(72) Erfinder: Plötz, Josef, Dr., 64625 Bensheim (DE)

(57) **Zusammenfassung**

Ein solches Röntgendiagnostikgerät weist einen Strahlensender (1) und ein Referenzobjekt (2) auf, wobei gemäß der Erfindung das Referenzobjekt (2) einen festen Bezug zum Strahlensender (1) hat, mit dem es ausschließlich verbunden ist. Es ist somit möglich, den Abstand des Strahlensenders (1) zum Strahlenempfänger (13) und zum Untersuchungsobjekt den Strahlungswinkel und die Durchstrahlungsrichtung aufgrund der beim Durchstrahlen des Referenzobjektes (2) vom Strahlenempfänger (13) ableitbaren Signale zu bestimmen, ohne daß hierbei das Referenzobjekt (2) im Bereich des Untersuchungsobjektes stört.

## Beschreibung

Aus der WO 93/22 893 A1 ist eine Methode bekannt, mit der es möglich ist, eine Aufnahme eines Untersuchungsobjektes zu rekonstruieren, ohne daß hierbei die Projektionswinkel α und die geometrische Anordnung von Strahlensender und Strahlenempfänger und die Fokalebene bekannt sind. Gemäß dieser Methode ist im Bereich des Strahlenempfängers eine Referenz aus strahlenabsorbierendem Material mit bekannter Größe und bekanntem Abstand zum Strahlenempfänger vorgesehen, die bei jeder Einzelprojektion auf den Strahlenempfänger projeziert wird. Aufgrund der örtlichen Abbildung der Referenz auf dem Strahlenempfänger für jede Einzelprojektion kann die geometrische Anordnung und der zweidimensionale Projektionswinkel α ermittelt werden.

Eine Halterung zum Positionieren eines Strahlensenders eines Röntgendiagnostikgeräts für Tomosynthese ist aus der DE 44 14 689 A1 bekannt. An die Halterung ist ein Tragarm angekoppelt, an dem - in Strahlungsrichtung gesehen - vor dem Untersuchungsobjekt ein kugelförmiges Referenzobjekt und hinter dem Untersuchungsobjekt ein Strahlenempfänger angeordnet sind. Über die Halterung wird zum einen der Abstand des Strahlensenders zum Referenzobjekt und zum Strahlenempfänger sowie der Winkel α eines vom Strahlensender emittierten Strahlenbündels zu einer Bezugsachse der Haltevorrichtung vorgegeben. Es ist ferner bekannt, die Strahlenquelle verstellbar in einem Gehäuse anzuordnen, an das eine Positionierungsvorrichtung für das Referenzobjekt und den Strahlenempfänger ankoppelbar ist.

Aufgabe der Erfindung ist die weitere vorteilhafte Ausgestaltung des Röntgendiagnostikgerätes der eingangs genannten Art.

Die Aufgabe wird erfindungsgemäß durch den Gegenstand des Patentanspruches 1 gelöst.

Vorteil der Erfindung ist, daß das Referenzobjekt nicht mehr Objekt nah angeordnet ist, so daß es bei der Positionierung nicht mehr stört oder behindert.

Es ist besonders vorteilhaft, wenn das Referenzobjekt aus mehreren Teilobjekten besteht, die balkenförmig ausgebildet sind und die sich kreuzen. Durch die Projektion des Referenzobjektes auf den Strahlenempfänger und Auswerten der Signale, die vom Referenzobjekt erzeugt werden, kann sowohl der Abstand der Strahlensender zum Strahlenempfänger, der Einstrahlungswinkel (Projektionswinkel) und die Einstrahlungsrichtung bestimmt werden. Es ist somit möglich, Daten zur Berechnung einer Tomosyntheseaufnahme aus den beim Durchstrahlen des Untersuchungsobjektes aus unterschiedlichen Richtungen gewonnenen und vom Strahlenempfänger abgeleiteten Bildsignalen zu erhalten. Alternativ kann das Referenzobjekt hierzu auch von wenigstens drei Teilobjekten gebildet sein, deren Anordnung zueinander von einer Geraden abweicht. Von besonderem Vorteil ist es, wenn sich die Teilobjekte oder sich Bereiche der Teilobjekte hinsichtlich der Strahlenabsorption unterscheiden, so daß sich der Abstand, der Einstrahlungswinkel und die Einstrahlungsrichtung in eindeutiger Weise bestimmen lassen.

Weitere Vorteile und Einzelheiten der Erfindung ergeben sich aus der nachfolgenden Beschreibung eines Ausführungsbeispieles anhand der Zeichnung in Verbindung mit den Unteransprüchen.

Es zeigt:
- FIG 1: ein Röntgendiagnostikgerät für Tomosynthese in prinzipieller Darstellung, wobei das Referenzobjekt einen festen Bezug zur Strahlenquelle hat,
- FIG 2: ein erstes und

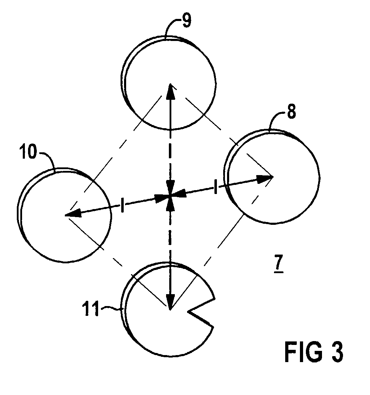

- FIG 3: ein Zweites Ausführungsbeispiels eines Referenzobjektes des Röntgendiagnostikgerätes nach FIG 1.

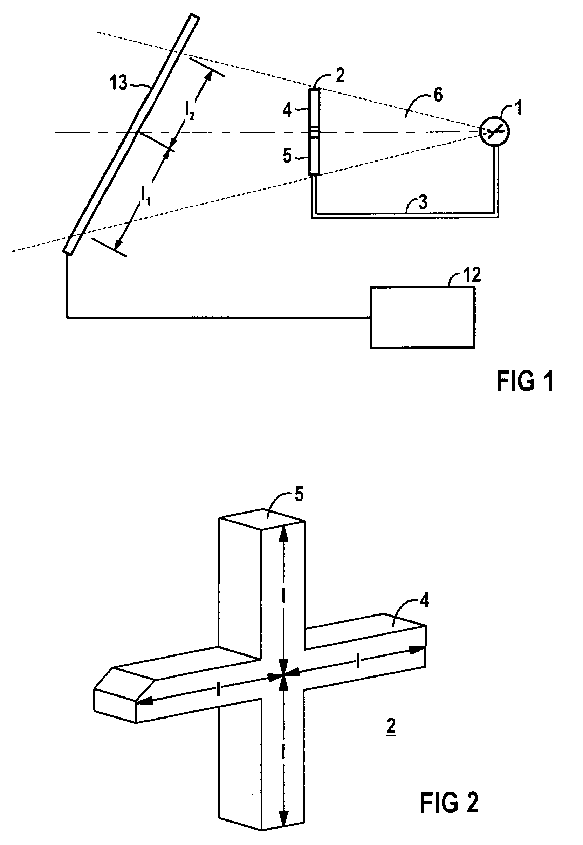

In der FIG 1 ist mit dem Bezugszeichen 1 ein Strahlensender, insbesondere eine Strahlenquelle gekennzeichnet zu dem / der ein Referenzobjekt 2 einen festen Bezug hat und mit dem / der es ausschließlich verbunden ist. Das Referenzobjekt 2 kann hierzu beispielsweise über eine Halterung 3 ausschließlich mit dem Strahlensender 1 gekoppelt sein. Das vorzugsweise aus wenigstens zwei Teilobjekten 4,5 bestehende Referenzobjekt 2 ist hierbei so angeordnet, daß es im Strahlenbündel 6 liegt, das von dem Strahlensender 1 ausgeht. Im Ausführungsbeispiel sind die Teilobjekte 4,5 balkenförmig ausgebildet und kreuzen sich, was insbesondere aus der FIG 2 hervorgeht. Die Länge 1 der Balkenabschnitte als Teilbereiche der Teilobjekte 4,5 kann gleich oder unterschiedlich sein. Vorzugsweise sind die Teilobjekte 4,5, aber hinsichtlich der Strahlenabsorption unterschiedlich ausgebildet, so daß auch die Einstrahlungsrichtung in eindeutiger Weise bestimmt werden kann, während der Abstand zum Strahlenempfänger 13 und der Einstrahlungswinkel (dem Betrage nach) bereits ermittelt werden können, wenn die Längen oder Abmessungen der Teilbereiche der Teilobjekte 4,5 bekannt sind. Die Unterscheidung hinsichtlich der Strahlenabsorption kann hierbei dadurch bewirkt werden, daß Teilbereiche der Teilobjekte 4,5 sich hinsichtlich der Länge und/oder der Form oder in sonstiger Weise unterscheiden. Es ist auch möglich, nur einen Teilbereich eines Teilobjektes 4,5 hinsichtlich der Strahlenabsorption unterschiedlich zu den anderen Teilbereichen auszuführen.

Bei einem alternativen Referenzobjekt 7 sind wenigstens drei Teilobjekte 8,9,10 und im Ausführungsbeispiel ein weiteres Teilobjekt 11 vorgesehen, deren Anordnung zueinander von einer Geraden abweicht. Diese Teilobjekte 8,9,10,11 bilden einen geometrischen Körper, beispielsweise ein Mehreck, wobei die Abstände der Teilobjekte 8,9,10,11 vorgegeben sind. In diesem Ausführungsbeispiel sind die Teilobjekte 8,9,10,11 als Scheiben ausgebildet, sie können aber auch kugelförmig sein oder auch eine andere Form annehmen. Zur eindeutigen Bestimmung der Einstrahlungsrichtung kann sich zumindest ein Teilobjekt 11 hinsichtlich der Form und/oder der Strahlenabsorption gegenüber den weiteren Teilobjekten 8,9,10 unterscheiden.

Alle beschriebenen Referenzobjekte und/oder Teilobjekte können auch in invertierter Form ausgeführt sein, d.h. als Ausnehmungen in vollem Material. Vorzugsweise wird man scheibenförmiges Vollmaterial wählen mit Außenabmessungen, die nicht wesentlich größer als das Referenzobjekt selbst sind, und die Ausnehmungen werden ganz oder fast ganz durch die Scheibe gehen.

Einer Auswerteeinrichtung 12 werden die Signale eines Strahlenempfängers 13 zugeführt, so daß aus den durch die Projektion des Referenzobjektes 2 auf den Strahlenempfänger 13 ableitbaren Signalen der Abstand, der Einstrahlungswinkel und die Einstrahlungsrichtung bestimmt werden können. Befindet sich ein Untersuchungsobjekt - aus Richtung des Strahlensenders 1 gesehen - vor dem Strahlenempfänger 13 und wird dieses aus unterschiedlichen Richtungen durchstrahlt, so kann aufgrund der aus jeder Durchstrahlungsrichtung erhaltenen Signale des Untersuchungsobjektes sowie den Signalen des Referenzobjektes eine Tomosyntheseaufnahme des Untersuchungsobjektes erstellt werden.

## Patentansprüche

1. Röntgendiagnostikgerät für Tomosynthese mit einem Strahlensender (1) und einem Referenzobjekt (2),
wobei das Referenzobjekt (2) einen festen Bezug zum Strahlensender(1) hat, mit dem es ausschließlich verbunden ist.

2. Röntgendiagnostikgerät nach Anspruch 1,
wobei das Referenzobjekt (2) von wenigstens zwei Teilobjekten (4,5,8,9,10,11) gebildet ist.

3. Röntgendiagnostikgerät nach Anspruch 2,
wobei die Teilobjekte (4,5) balkenförmig ausgebildet sind und sich kreuzen.

4. Röntgendiagnostikgerät nach Anspruch 2,
wobei das Referenzobjekt (2) von wenigstens drei Teilobjekten (8,9,10,11) gebildet ist, deren Anordnung zueinander von einer Geraden abweicht.

5. Röntgendiagnostikgerät nach einem der Ansprüche 2 bis 4,
**dadurch gekennzeichnet,**
daß die Teilobjekte (4,5,8,9,10,11) einen geometrischen Körper bilden.

6. Röntgendiagnostikgerät nach Anspruch 5,
wobei die Teilobjekte (8,9,10,11) ein Mehreck bilden.

7. Röntgendiagnostikgerät nach einem der Ansprüche 4 bis 6,
wobei die Teilobjekte (8,9,10,11) als Scheiben oder Kugeln ausgebildet sind.

8. Röntgendiagnostikgerät nach einem der Ansprüche 2 bis 7,
wobei sich wenigstens zwei Teilobjekte (4,5,8,9,10,11) hinsichtlich der Strahlenabsorption unterscheiden.

9. Röntgendiagnostikgerät nach einem der Ansprüche 1 bis 8,
wobei das Referenzobjekt (2) aus Strahlung absorbierendem Material besteht, in dem Bereiche geringerer Strahlenabsorption ausgebildet sind.
